# EUROPEAN PATENT APPLICATION

(11) **EP 4 708 325 A1**
(43) Date of publication of application: **11.03.2026**
(21) Application number: 24797527.9
(22) Date of filing: 15.03.2024
(51) Int. Cl.: G16H 50/70, G16H 50/30, G16H 10/60, G16H 10/40, G16H 70/00, G16H 15/00, G16B 25/00, G16B 40/10, G16H 20/10, G16H 80/00

(54) **METHOD AND APPARATUS FOR PROVIDING SIMILARITY CASE FOR PERSON TO BE TREATED BY USING MDX DATA**

(30) Priority: 26.04.2023 KR 20230054400
(71) Applicant: Seegene, Inc., Seoul 05548 (KR)
(72) Inventor: KIM, Young Wook, Seoul 05548 (KR); JEON, Chang Ha, Seoul 05548 (KR)
(74) Representative: Viering, Jentschura & Partner mbB Patent- und Rechtsanwälte
(86) International application number: PCT/KR2024/095579
(87) International publication number: WO 2024/225875

(57) **Abstract**

A method for providing a similarity case of a target patient using molecular diagnostic (MDx) data, performed by an apparatus for providing the similarity case of the target patient, includes: obtaining reference MDx data for MDx data of the target patient by comparing the MDx data of the target patient with a previously stored set of MDx data; obtaining an electronic medical record (EMR) of a reference patient(s) corresponding to health information of the target patient by comparing the health information of the target patient with health information of the reference patient from whom the reference MDx data are obtained; and providing the obtained EMR and the obtained reference MDx data as the similarity case to a healthcare provider's terminal.

## Description

### TECHNICAL FIELD

The present invention relates to an apparatus for and method of providing a similarity case.

### BACKGROUND ART

Molecular diagnostic (MDx) data (hereinafter, MDx data) refers to various data that may be obtained from diagnostics that detect changes at the molecular level through numerical values or images, and may be data obtainable from in vitro or in vivo diagnostics. MDx data includes data obtained from DNA or RNA nucleic acid analysis, protein analysis, and intracellular metabolome analysis.

MDx data has been a means usefully used to determine a disease in a healthcare provider's diagnosis process. Conventional MDx data was used as reference material as an auxiliary means for a healthcare provider and did not directly affect the healthcare provider's decision or judgment on treatment methods. That is, the healthcare provider only referred to the MDx data during the diagnosis process and mainly used clinical methods that rely on their experience and knowledge to determine treatment methods in the treatment process, failing to effectively utilize the MDx data used for diagnosis in the treatment process.

However, since MDx data may appear differently depending on the patient's disease symptoms, the course of recovery, and the patient's condition, it statistically contains various information about the patient and may provide meaningful information that may be utilized in the treatment process. In other words, although MDx data is key information that may determine the patient's current state and treatment method, there is currently no method to utilize it effectively.

### DETAILED DESCRIPTION OF THE INVENTION

### TECHNICAL PROBLEMS

A problem to be solved described in the present specification is to provide a method and apparatus for providing a similarity case of a target patient to a healthcare provider's terminal.

Another problem to be solved described in the present specification is to provide a similarity case method and apparatus that allows a healthcare provider to utilize an electronic medical record and retrieved reference MDx.

Yet another problem to be solved described in the present specification is to provide a similarity case method and apparatus that may determine the diagnosis and treatment method for a target patient through past data similar to the target patient.

However, the problems to be solved described in the present specification are not limited to the above-mentioned problems, and other unmentioned problems will be clearly understood by those of ordinary skill in the art to which the present invention pertains from the following description.

### MEANS FOR SOLVING PROBLEMS

A method for providing a similarity case of a target patient using molecular diagnostic (MDx) data performed by an apparatus for providing the similarity case of the target patient according to an embodiment may include: obtaining reference MDx data for MDx data of the target patient by comparing the MDx data of the target patient with a previously stored set of MDx data; obtaining an electronic medical record (EMR) of a reference patient corresponding to health information of the target patient by comparing the health information of the target patient with health information of the reference patient from whom the reference MDx data are obtained; and providing the obtained EMR and the obtained reference MDx data as the similarity case to a healthcare provider's terminal.

In one embodiment, the comparison of the MDx data of the target patient with the previously stored set of MDx data may be performed by using a laboratory information system (LIS) storing the MDx data.

In one embodiment, the obtainment of the EMR may be performed by using an EMR system storing the EMR.

In one embodiment, the MDx data may be obtained from a plurality of target nucleic acid amplification reactions performed to detect a plurality of target nucleic acids of interest in a sample.

In one embodiment, the MDx data may include at least one of positive/negative determination results for the plurality of target nucleic acids of interest, Ct values for the plurality of target nucleic acids of interest, a dataset obtained from amplification reactions of the plurality of target nucleic acids of interest, and a value of a parameter indicating an aspect of the dataset.

In one embodiment, the reference MDx data may be obtained by using at least one identical element among a reagent, an equipment, and an implementation method for the plurality of target nucleic acid amplification reactions for obtaining the MDx data.

In one embodiment, the identical equipment may include at least one selected from the group consisting of a nucleic acid extraction device, a liquid handler, and a real-time amplification device.

In one embodiment, the equipment may have an identical calibration applied.

In one embodiment, the health information may be extracted from content included in at least one of an EMR, a personal health record, and a health examination record.

In one embodiment, the health information may include at least one of a symptom, an age, a gender, a vaccination history, a medication information, an underlying disease, and a lifestyle habit.

In one embodiment, the electronic medical record provided to the healthcare provider's terminal may include at least one of a symptom, an age, a gender, a vaccination history, a medication information, an underlying disease name, and a lifestyle habit, the electronic medical record may be exclusive of predefined sensitive information from health information included in the EMR, and the predefined sensitive information may include personal information.

In one embodiment, the reference patient corresponding to the health information of the target patient may be selected by comparing at least one of the following between the target patient and the reference patient: a symptom, an age, a gender, a vaccination history, a medication information, an underlying disease name, and a lifestyle habit.

In one embodiment, said providing the obtained EMR and the obtained reference MDx data as the similarity case to the healthcare provider's terminal may include: providing, to the healthcare provider's terminal, information about a compared item between the EMR of the reference patient and the EMR of the target patient, together with the similarity case.

In one embodiment, said providing the obtained EMR and the obtained reference MDx data as the similarity case to the healthcare provider's terminal may include: providing, to the healthcare provider's terminal as the similarity case, a symptom of the reference patient, MDx data-based treatment information and post-treatment outcome information.

An apparatus for providing the similarity case of a target patient according to another embodiment may include: a processor; a network interface; a memory loading a computer program when executed by the processor; and a storage storing the computer program, wherein the computer program includes: an instruction for obtaining reference MDx data for MDx data of the target patient by comparing the MDx data of the target patient with a previously stored set of MDx data; an instruction for obtaining an electronic medical record (EMR) of a reference patient(s) corresponding to health information of the target patient by comparing the health information of the target patient with health information of the reference patient from whom the reference MDx data are obtained; and an instruction for providing the obtained EMR and the obtained reference MDx data as the similarity case to a healthcare provider's terminal.

In one embodiment, the MDx data may be obtained from a plurality of target nucleic acid amplification reactions performed to detect a plurality of target nucleic acids of interest in a sample.

In one embodiment, the MDx data may include at least one of positive/negative determination results for the plurality of target nucleic acids of interest, Ct values for the plurality of target nucleic acids of interest, a dataset obtained from amplification reactions of the plurality of target nucleic acids of interest, and a value of a parameter indicating an aspect of the dataset.

In one embodiment, the reference MDx data may be obtained by using at least one identical element among a reagent, an equipment, and an implementation method for the plurality of target nucleic acid amplification reactions for obtaining the MDx data.

In one embodiment, the health information may be extracted from content included in at least one of an electronic medical record, a personal health record, and a health examination record.

In one embodiment, the health information may include at least one of a symptom, an age, a gender, a vaccination history, a medication information, an underlying disease, and a lifestyle habit.

A computer-readable recording medium storing a computer program according to another embodiment, wherein the computer program may be programmed to perform: obtaining reference MDx data for MDx data of a target patient by comparing the MDx data of the target patient with a previously stored set of MDx data; obtaining an electronic medical record (EMR) of a reference patient(s) corresponding to health information of the patient by comparing the health information of the target patient with health information of the reference patient from whom the reference MDx data are obtained; and providing the obtained EMR and the obtained reference MDx data as a similarity case of the target patient to a healthcare provider's terminal.

A computer program stored in a computer-readable recording medium according to another embodiment, wherein the computer program may be programmed to perform: obtaining reference MDx data for MDx data of a target patient by comparing the MDx data of the target patient with a previously stored set of MDx data; obtaining an electronic medical record (EMR) of a reference patient(s) corresponding to health information of the target patient by comparing the health information of the target patient with health information of the reference patient from whom the reference MDx data are obtained; and providing the obtained EMR and the obtained reference MDx data as a similarity case of the target patient to a healthcare provider's terminal.

### EFFECTS OF THE INVENTION

According to an embodiment, a similarity case that may be used for the diagnosis and treatment of a target patient may be provided to a healthcare provider's terminal.

According to an embodiment, objective information for determining the current state of a target patient may be provided.

According to an embodiment, a suitable treatment method and the degree of future improvement for a target patient may be predicted and determined.

According to an embodiment, by effectively utilizing electronic medical records and MDx data, a suitable treatment precedent for a target patient may be provided to a healthcare provider.

According to an embodiment, an objective and highly accurate diagnosis and a proven treatment method may be proposed by using objective data on symptoms and treatment methods according to the target patient's MDx data.

### BRIEF DESCRIPTION OF THE DRAWINGS

FIG. 1 is a diagram conceptually illustrating a relationship between an apparatus for providing a similarity case of a target patient using MDx data and a healthcare provider's terminal according to an embodiment.
FIG. 2 is a flowchart of a method for providing a similarity case of a target patient using MDx data according to an embodiment.
FIG. 3 is a diagram illustrating an example of molecular diagnostic (MDx) data.
FIG. 4 is a flowchart specifically illustrating an operation of retrieving reference MDx data with an MDx similarity score equal to or greater than a predetermined MDx similarity score.
FIG. 5 is a diagram for explaining MDx data with an MDx similarity score equal to or greater than a predetermined MDx similarity score.
FIG. 6 is a diagram for explaining MDx data with an MDx similarity score less than a predetermined MDx similarity score.
FIG. 7 is a flowchart specifically illustrating an operation of selecting a patient with a health similarity score equal to or greater than a predetermined health similarity score by comparing health information of a reference patient corresponding to reference MDx data with health information of a target patient.
FIG. 8 is an exemplary diagram showing health information of patients using various items.
FIG. 9 is a diagram for explaining a structure in which the apparatus for providing a similarity case of a target patient using MDx data of FIG. 1 operates using an LIS server and an EMR system.
FIG. 10 is a hardware configuration diagram of an apparatus for providing a similarity case of a target patient using MDx data.

### MODE FOR IMPLEMENTING THE INVENTION

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

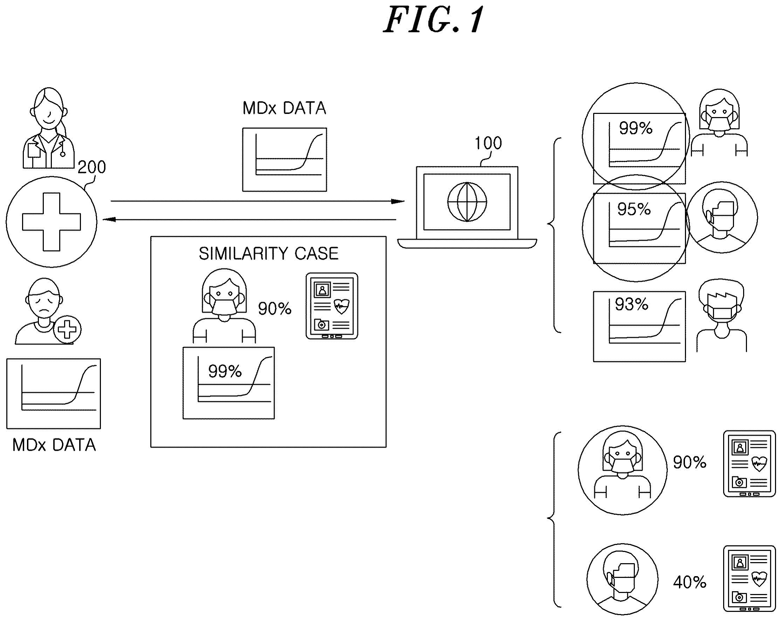

Before describing FIG. 1, terms used herein will be reviewed.

The term "sample" includes biological samples (e.g., cells, tissues, and body fluids) and non-biological samples (e.g., food, water, and soil). Among these, the biological sample may include, for example, at least one of a virus, bacterium, tissue, cell, blood (including whole blood, plasma, and serum), lymph, bone marrow fluid, saliva, sputum, swab, aspiration fluid, milk, urine, stool, ocular fluid, semen, brain extract, cerebrospinal fluid, joint fluid, thymus fluid, bronchial lavage fluid, ascites, and amniotic fluid. Such samples may or may not contain the aforementioned target analyte.

Meanwhile, when the aforementioned target analyte is a nucleic acid molecule or contains a nucleic acid molecule, a nucleic acid extraction process known in the art may be performed on the sample presumed to contain the target analyte (see: Sambrook, J. et al., Molecular Cloning. A Laboratory Manual, 3rd ed. Cold Spring Harbor Press (2001)). The nucleic acid extraction process may vary depending on the type of sample. Furthermore, when the extracted nucleic acid is RNA, a reverse transcription process to synthesize cDNA may be additionally performed (see: Sambrook, J. et al., Molecular Cloning. A Laboratory Manual, 3rd ed. Cold Spring Harbor Press (2001)).

The term "target" includes various substances (e.g., biological substances and non-biological substances), which may refer to the same object as the term "target analyte" or "target substance".

Specifically, this target may include at least one of a biological substance, more specifically a nucleic acid molecule (e.g., DNA and RNA), protein, peptide, carbohydrate, lipid, amino acid, biological compound, hormone, antibody, antigen, metabolite, and cell. The target may include a target nucleic acid molecule.

The term "cycle" refers to a unit of change in the conditions in a plurality of measurements involving a change in certain conditions. The change in the certain conditions means an increase or decrease in, for example, temperature, reaction time, number of reactions, concentration, pH, number of replications of the measurement target (e.g., nucleic acid), and the like. Therefore, a cycle may be a time or process cycle, a unit operation cycle, and a reproductive cycle.

More specifically, the term "cycle" refers to one unit of repetition when a reaction of a certain process is repeated or when a reaction is repeated based on a certain time interval.

Alternatively, the term "cycle" may refer to one unit of repetition when a certain action is repeated as a reaction progresses.

For example, when a nucleic acid amplification reaction is performed, the act of detecting a signal generated at regular time intervals may be repeated, and one unit of the repetition may be referred to. In this case, the cycle may have a unit of time.

For example, in the case of a nucleic acid amplification reaction, one cycle means a reaction including a denaturation step of the nucleic acid, an annealing step of the primer, and an extension step of the primer. In this case, the change in a certain condition is an increase in the number of reaction repetitions, and the repetition unit of the reaction including the series of steps is set as one cycle. The number of cycles may include the number of reactions or the reaction time.

Meanwhile, an amplification reaction for amplifying a signal indicating the presence of a target (target analyte or target of interest) may be performed in a way that the signal is amplified as the target is amplified (for example, a real-time PCR method). Alternatively, according to one embodiment, the amplification reaction may be performed in a way that only the signal indicating the presence of the target is amplified without the target being amplified (for example, CPT method (Duck P, et al., Biotechniques, 9:142-148(1990)), Invader assay (U.S. Patent Nos. 6,358,691 and 6,194,149)).

Meanwhile, the aforementioned target or target analyte, particularly the target nucleic acid molecule, may be amplified by various methods: polymerase chain reaction (PCR), ligase chain reaction (LCR) (U.S. Patent Nos. 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al., J. Clin. Microbiol. 33:1856-1859 (1995)), nucleic acid sequence-based amplification (NASBA) (Compton, Nature 350(6313):91-2 (1991)), rolling circle amplification (RCA) (Lisby, Mol. Biotechnol. 12(1):75-99 (1999); Hatch et al., Genet. Anal. 15(2):35-40 (1999)), Q-Beta Replicase (Lizardi et al., BiolTechnology 6:1197(1988)), loop-mediated isothermal amplification (LAMP, Y. Mori, H. Kanda and T. Notomi, J. Infect. Chemother., 2013, 19, 404-411), and recombinase polymerase amplification (RPA, J. Li, J. Macdonald and F. von Stetten, Analyst, 2018, 144, 31-67).

Meanwhile, the amplification reaction amplifies a signal accompanied by the amplification of the target (specifically, the target nucleic acid molecule). For example, the amplification reaction is performed according to PCR, specifically real-time PCR, or by an isothermal amplification reaction (for example, LAMP or RPA).

In the present specification, the term "signal value" refers to a value obtained by quantifying the level (e.g., intensity of the signal) of a signal actually measured in a cycle of a signal-generating reaction, particularly an amplification reaction, according to a certain scale, or a modified value thereof. The modified value may include a mathematically processed signal value of the actually measured signal value. Examples of mathematically processed signal values of the actually measured signal value (i.e., the signal value of the raw dataset) may include a value obtained by adding, multiplying, subtracting, or dividing the measured signal value by a selected constant, a logarithmic value, or a value of change.

According to one embodiment of the present invention, a signal value refers to a value that absolutely or relatively quantifies the magnitude of the signal first detected by a detector at the corresponding cycle number. Although the signal value may include a change value that considers the relationship with other cycles, it is preferable to use the signal value excluding the change value when calculating the first-order change value in the method of an embodiment. The signal value used in the first step of the method of an embodiment is also called a "zeroth-order signal value," a "raw signal value," or an "original signal value" in comparison with the first-order change value and the second-order change value. The unit of the signal value may vary depending on the type of signal generation reaction used. For example, when a signal value is obtained in each cycle by a real-time PCR amplification reaction, the signal value may be a value expressed in RFU (Relative Fluorescence Unit).

The term "target presence/absence determination process" refers to an operation of determining the presence or absence of a target in a sample using signal values, and additionally includes operations of analyzing the data used to determine the presence or absence of the target.

FIG. 1 is a diagram conceptually illustrating a relationship between an apparatus for providing a similarity case of a patient using MDx data 100 and a healthcare provider's terminal 200 according to one embodiment.

Referring to FIG. 1, the apparatus for providing a similarity case of a target patient using MDx data 100 (hereinafter, 'apparatus for providing a similarity case 100') may provide a similarity case of the target patient to a healthcare provider's terminal 200 using molecular diagnostic (MDx) data.

In the present specification, the molecular diagnostic (MDx) data refers to various data that may be obtained from diagnostics that detect changes at the molecular level through numerical values or images, and may be data obtainable from in vitro or in vivo diagnostics. In the embodiment, the MDx data includes DNA or RNA nucleic acid analysis, protein analysis, and intracellular metabolome (metabolome) analysis. The MDx data may be obtained from a plurality of target nucleic acid amplification reactions performed to detect a plurality of target nucleic acids of interest in a sample.

The apparatus for providing the similarity case 100 may obtain the MDx data from the healthcare provider's terminal 200 and retrieve reference MDx data using the obtained MDx data.

In this regard, the apparatus for providing the similarity case 100 may directly obtain the MDx data of the target patient from the healthcare provider's terminal 200, or may obtain only the identification information of the target patient (e.g., the target patient's id) from the healthcare provider's terminal 200 and obtain the MDx data from an external server using the obtained identification information. The method by which the apparatus for providing the similarity case 100 obtains the MDx data is not limited thereto.

The apparatus for providing the similarity case 100 may retrieve reference MDx data with an MDx similarity score equal to or greater than a predetermined MDx similarity score by comparing the MDx data of the target patient with previously stored MDx data.

At this time, the apparatus for providing the similarity case 100 may retrieve reference MDx data with the MDx similarity score equal to or greater than the predetermined MDx similarity score by comparing the MDx data of the target patient with previously stored MDx data.

The apparatus for providing the similarity case 100 may obtain an electronic medical record (EMR) of the patient corresponding to the patient's health information by comparing the health information of the reference patient from whom the retrieved reference MDx data was obtained with the health information of the target patient.

At this time, when comparing the health information of the reference patient from whom the retrieved reference MDx data was obtained with the health information of the target patient, the apparatus for providing the similarity case 100 may select the patient with a health similarity score equal to or greater than a predetermined health similarity score.

The apparatus for providing the similarity case 100 may provide the obtained electronic medical record and the obtained reference MDx data as the similarity case of the target patient to the healthcare provider's terminal 200.

Hereinafter, operations will be described with a focus on the healthcare provider's terminal 200.

The healthcare provider's terminal 200 may transmit the MDx data of the target patient to the apparatus for providing the similarity case 100 or request the similarity case from the apparatus for providing the similarity case 100 using the target patient's identification ID.

Thereafter, the healthcare provider's terminal 200 may obtain the similarity case for the target patient from the apparatus for providing the similarity case 100. At this time, the healthcare provider's terminal 200 may be provided with the similarity case of 80%~100% for the target patient.

In one embodiment, a weight may be assigned to a desired item in the healthcare provider's terminal 200, and the healthcare provider's terminal 200 may obtain the similarity case reflecting the weight. For example, a weight may be assigned to any one of the items included in the MDx data in the healthcare provider's terminal 200, or a weight may be assigned to the health information of the target patient. For example, in the healthcare provider's terminal 200, a weight may be assigned to the Ct value of the MDx data, a weight may be assigned to each symptom of the target patient, or a weight may be assigned to the healthcare provider's gender, lifestyle habits, etc. Thereafter, the apparatus for providing the similarity case 100 may provide the similarity case reflecting the weight to the healthcare provider's terminal 200.

In one embodiment, the healthcare provider's terminal 200 may be provided with data listing the possibilities of expected diseases in order of rank along with the similarity case, and may be provided with medical guideline information such as treatment methods, treatment duration, post-treatment progress, treatment costs, emergency measures, diet, and recommended advanced medical institutions.

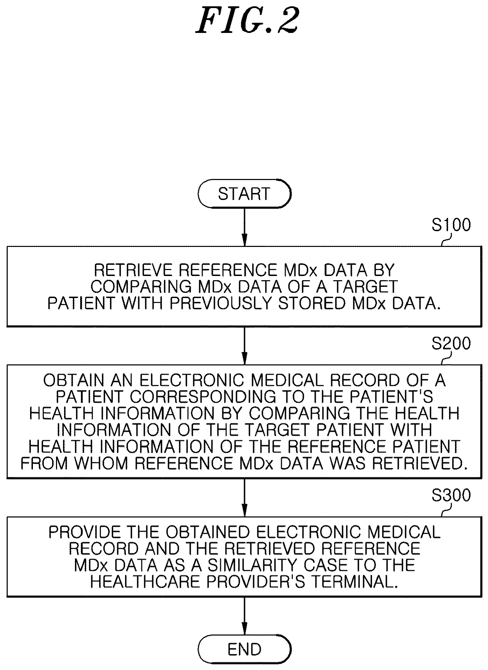

The operation of the apparatus for providing the similarity case 100 has been briefly reviewed in FIG. 1. Hereinafter, a method for providing a similarity case of a target patient using MDx data will be described with reference to FIGS. 2 to 7. FIGS. 2 to 7 may be performed by a computing apparatus. In one embodiment, the computing apparatus may be the apparatus for providing the similarity case 100 described in FIG. 1.

FIG. 2 is a flowchart of a method for providing the similarity case of a target patient using MDx data according to one embodiment.

Referring to FIG. 2, in step S100, MDx data of the target patient and previously stored MDx data may be compared to retrieve MDx data having similarity among the previously stored MDx data as reference MDx data.

Here, the previously stored MDx data may be pre-stored MDx data obtained from patients other than the target patient, or pre-stored MDx data obtained from the target patient, and there may be a plurality of such data.

Here, the MDx data having similarity may be determined as MDx data having similarity when a match is equal to or greater than a predetermined similarity by comparing elements included in the MDx data. The MDx data having similarity may be determined as reference MDx data. However, the method for retrieving the reference MDx data is not limited thereto.

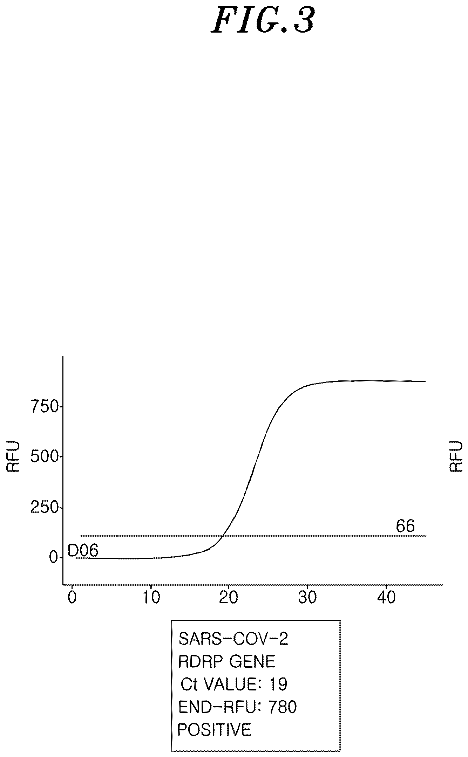

FIG. 3 is a diagram illustrating an example of molecular diagnostic (MDx) data.

The MDx data may be data obtained by performing a target presence/absence determination process using a dataset obtained from an amplification reaction of a target nucleic acid.

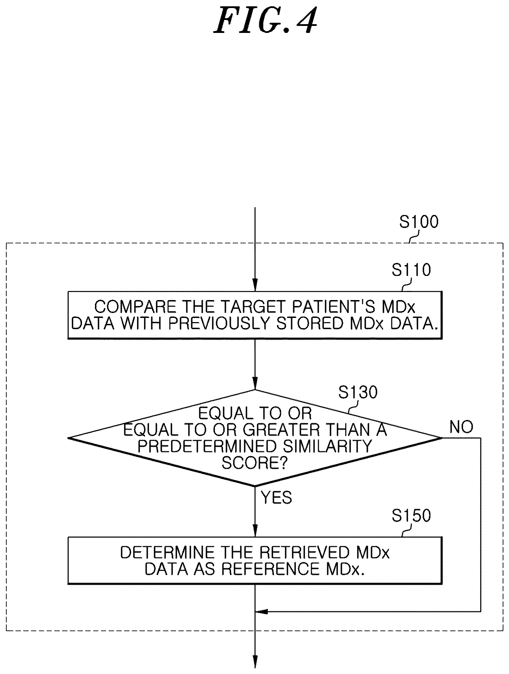

In one embodiment, the MDx data may be an amplification curve representing a signal value. The horizontal axis of the MDx data may be the number of cycles, and the vertical axis may be the RFU measured at each cycle. In FIG. 4, the target of the MDx data is the RdRP gene of SARS-CoV-2, the Ct value is 19, the End-RFU is 780, and the positive/negative determination result performed using the MDx data is Positive. FIGS. 4 to 6 will be described based on this MDx data.

FIG. 4 is a flowchart specifically illustrating an operation of retrieving the reference MDx data with the MDx similarity score equal to or greater than the predetermined MDx similarity score.

When the reference MDx data with the MDx similarity score equal to or greater than the predetermined MDx similarity score is retrieved by comparing the MDx data of the target patient with previously stored MDx data in step S100, the MDx data of the target patient and the previously stored MDx data may be compared in step S110.

In step S130, the MDx similarity score is calculated by comparing the results from the target patient's MDx data and the previously stored MDx data, and an operation may be performed to check whether the comparison result has similarity based on the calculated MDx similarity score.

Specifically, in step S110, data from performing a presence/absence determination process for the same target nucleic acid of the same analyte in the target patient's MDx data and the previously stored MDx data may be compared. At this time, the data from performing the presence/absence determination process may be any one of a positive/negative determination result for the target nucleic acid, a Ct value, and a final reaction signal value. Furthermore, in step S130, when the data from performing the presence/absence determination process is compared, any one of the positive/negative determination result for the target nucleic acid, the Ct value, and the final reaction signal value may be compared with each other.

In one embodiment, the MDx data may include at least one of positive/negative determination results for a plurality of target nucleic acids of interest, Ct values, a dataset obtained from amplification reactions of the plurality of target nucleic acids of interest, and a value of a parameter indicating an aspect of the dataset.

In this regard, the target patient's MDx data and the previously stored MDx data may be compared if they are data from performing a presence/absence determination process for the same target nucleic acid of the same analyte, and at least one of the positive/negative determination result, the Ct value, and the final reaction signal value for the target nucleic acid in the patient's MDx data and the previously stored MDx data may be compared with each other.

In step S130, it may be determined whether the similarity is equal to or greater than the predetermined MDx similarity score as the target patient's MDx and the previously stored MDx data are respectively compared. In one embodiment, in step S130, the MDx similarity score may be calculated to determine whether the similarity is equal to or greater than the predetermined MDx similarity score as the target patient's MDx and the previously stored MDx data are respectively compared. The operation of calculating the MDx similarity score may be performed according to a pre-implemented algorithm. At this time, the similarity may be calculated based on how similar the patient's MDx and the previously stored MDx data are for each item. At this time, different weights may be assigned to each item. For example, when comparing the target patient's MDx and the previously stored MDx data, a higher similarity may be calculated when the value of an item with a high weight is within a predetermined range compared to when the value of an item with a low weight is within a predetermined range.

In this manner, a comprehensive MDx similarity score may be calculated by comparing the items included in the target patient's MDx and the previously stored MDx data, calculating a score for each item, and comprehensively calculating a score for the items. The MDx similarity score may be calculated as an arithmetic value from 0% to 100%. Using this MDx similarity score, the reference MDx data having similarity may be obtained.

Meanwhile, the MDx data may include brief profile information, and when the MDx similarity score is calculated, the result of comparing the brief profile information included in the target patient's MDx data and the previously stored MDx data may be reflected.

A predetermined MDx similarity score may be set in advance. When the target patient's MDx and the previously stored MDx data are equal to or greater than the predetermined MDx similarity score, the MDx data that is equal to or greater than the predetermined MDx similarity score may be determined as the reference MDx data in step S150. In one embodiment, the reference MDx data may be determined by filtering-in the previously stored MDx data in descending order of the MDx similarity score calculated by comparison with the target patient's MDx data.

However, in addition to this method, various algorithms for regression or classification of conventionally disclosed data, such as K-Nearest Neighbor, SVM (Support Vector Machine), Decision Tree, and Greedy algorithm, may be used to find MDx data similar to the target patient's MDx data among the previously stored MDx data, but it is not limited thereto.

In an embodiment, the reference MDx data may be obtained by using at least one identical element among a reagent, an equipment, and an implementation method for the plurality of target nucleic acid amplification reactions for obtaining the MDx data. At this time, the reagent, equipment, and implementation method for the plurality of target nucleic acid amplification reactions for obtaining the reference MDx data may be identical to those for the patient's MDx data. The reference MDx data may be data obtained using the same reagent product and the same MDx equipment as the target patient's MDx data.

Here, the identical reagent means the same reagent product. For example, the identical reagent may mean that primers and probes used in the reagent are identical. The composition of the reagent may include oligos and enzymes. In one embodiment, the identical reagent may mean that the oligos and enzymes are identical. In an embodiment, the identical reagent may be a reagent from the same company's product, but this does not mean to exclude products from other companies.

Here, the identical equipment may be at least one selected from the group consisting of a nucleic acid extraction device, a liquid handler, and a real-time amplification device. The real-time amplification device may be a PCR device, but is not limited thereto. In one embodiment, the identical equipment may have an identical calibration applied.

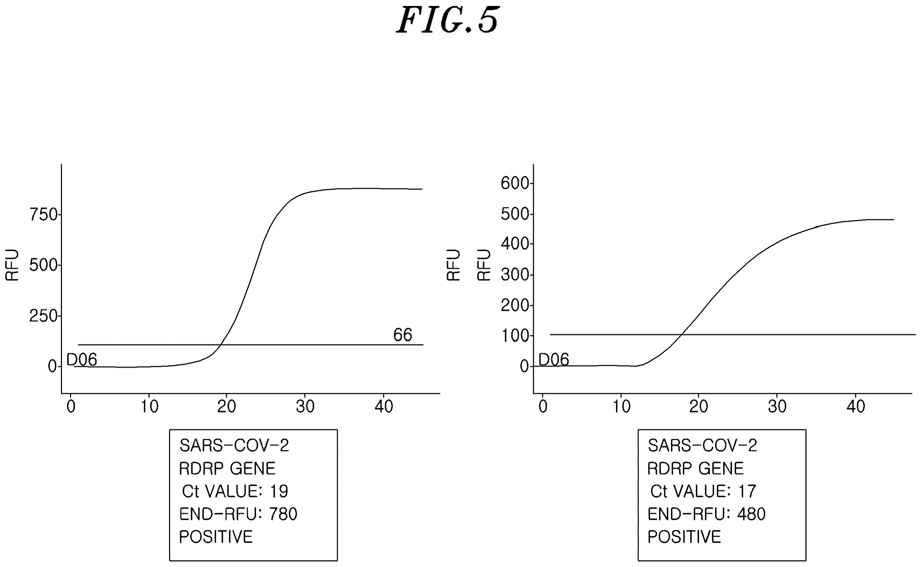

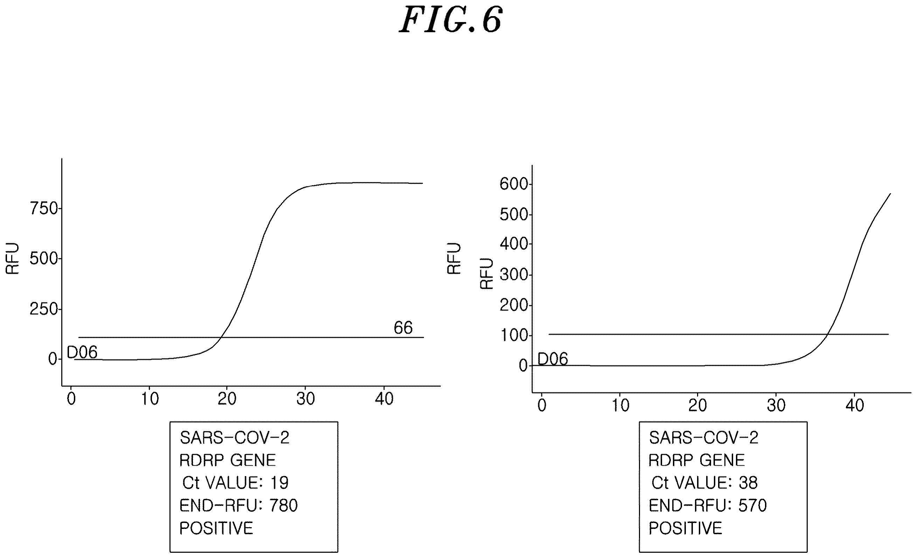

FIG. 5 is a diagram for explaining MDx data with the MDx similarity score equal to or greater than the predetermined MDx similarity score, and FIG. 6 is a diagram for explaining MDx data with the MDx similarity score less than the predetermined MDx similarity score.

The left MDx data in FIG. 5 is the data described in FIG. 3, which is the target patient's MDx data, and the right MDx data is an example of previously stored MDx data. The right MDx data targets the RdRP gene of SARS-CoV-2, has a Ct value of 17, an End-RFU of 480, and a presence/absence determination result of Positive.

When compared to the left MDx data, the right MDx data targets the RdRP gene of SARS-CoV-2, so it may correspond to the case of data from performing a presence/absence determination process for the same target nucleic acid of the same analyte.

Furthermore, when comparing the positive/negative determination result, Ct value, and final reaction signal value for the target nucleic acid of the right MDx data with those of the left MDx data, the Ct value differs by 2 cycles, the final reaction signal value, End-RFU, differs by 300, and the presence/absence determination result, which is the positive/negative determination result for the target nucleic acid, is the same at positive.

The MDx similarity score is calculated by comparing each item of the left MDx data and the right MDx data in FIG. 5, and when the MDx similarity score is equal to or greater than the predetermined MDx similarity score, the right MDx data of FIG. 5 may be determined as the reference MDx data. Since each item of the left MDx data and the right MDx data in FIG. 5 is similar, the right MDx data may be determined as the Reference MDx data for the target patient's MDx data.

Reference MDx data is MDx data that may be provided to the healthcare provider's terminal 200 and may be referred to for the target patient's treatment among the previously stored MDx data.

The left MDx data in FIG. 6 is the data described in FIG. 3, which is the target patient's MDx data, and the right MDx data is an example of previously stored MDx data. The right MDx data targets the RdRP gene of SARS-CoV-2, has a Ct value of 38, an End-RFU of 570, and a presence/absence determination result of Negative.

When compared to the left MDx data, the right MDx data targets the RdRP gene of SARS-CoV-2, so it may correspond to the case of data from performing a presence/absence determination process for the same target nucleic acid of the same analyte.

However, when comparing the positive/negative determination result, Ct value, and final reaction signal value for the target nucleic acid of the right MDx data with those of the left MDx data, the Ct value differs by 19 cycles, the final reaction signal value, End-RFU, differs by 200, and the presence/absence determination result, which is the positive/negative determination result for the target nucleic acid, is different at Negative.

The MDx similarity score is calculated by comparing each item of the left MDx data and the right MDx data in FIG. 6, and when the MDx similarity score is equal to or greater than the predetermined MDx similarity score, the right MDx data of FIG. 6 may be determined as the reference MDx data. Since most of the items of the left MDx data and the right MDx data in FIG. 6 are significantly different, the right MDx data is not determined as the Reference MDx data for the target patient's MDx data. However, this method is just one example, and it may be determined as Reference MDx data depending on the algorithm.

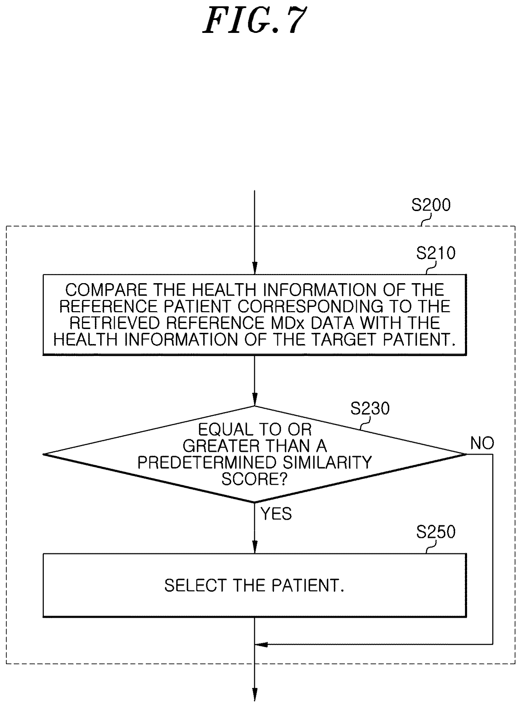

FIG. 7 is a flowchart specifically illustrating an operation of selecting a patient with a similarity of a predetermined level or higher by comparing the health information of a reference patient corresponding to the reference MDx data with the health information of the target patient.

The reference MDx data retrieved in step S100 may be one, but it may also be two or more. Furthermore, when two or more reference MDx data are retrieved in step S100, the reference MDx data may be data for one patient, but it may also be data for two or more patients.

Therefore, in this step, when the reference MDx data is for two or more patients, a patient with a similar health status or condition to the patient may be selected from among the two or more patients by comparing the health information of the two or more patients with the health information of the target patient.

Specifically, as shown in FIG. 7, when a patient with a health similarity score equal to or greater than the predetermined health similarity score is selected in step S200, the health information of the reference patient corresponding to the retrieved reference MDx data and the health information of the target patient may be compared in step S210.

At this time, the health information may be extracted from content included in at least one of an electronic medical record, a personal health record, and a health examination record. Health information may be extracted by collecting text included in at least one of an electronic medical record, a personal health record, and a health examination record using various conventionally disclosed algorithms.

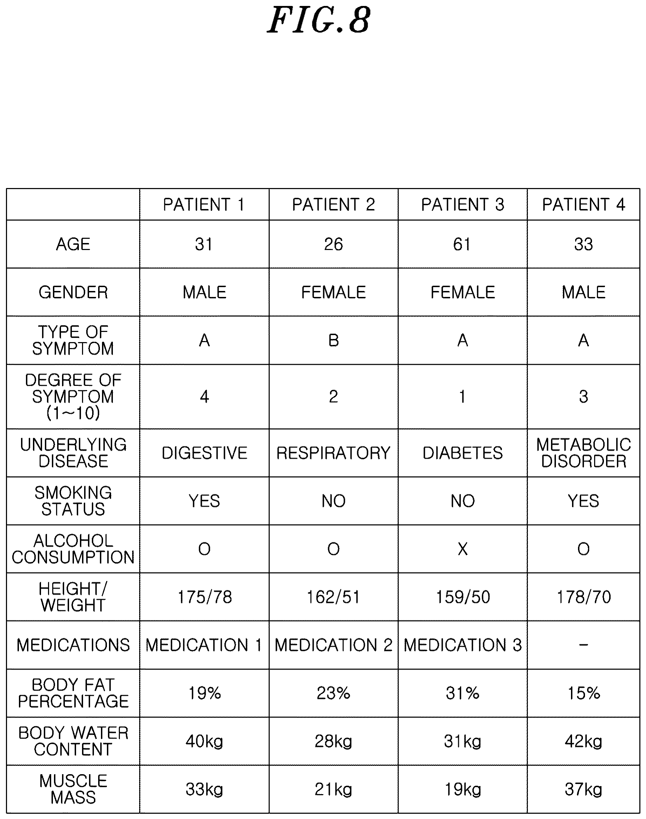

The health information may include 'age', 'gender', 'type of symptom', 'degree of symptom', 'underlying disease', 'smoking status', 'alcohol consumption', 'height/weight', 'medications', 'body fat percentage', 'body water content', and 'muscle mass' for each patient, as in the data referenced in FIG. 8. The health information shown in FIG. 8 is one example and is not limited thereto.

In step S210 of FIG. 7, when the health information of the reference patient corresponding to the retrieved reference MDx data and the health information of the target patient are compared, each item included in FIG. 8 may be compared with each other. For example, when comparing the health information of patient 1 and patient 2, a health similarity score may be calculated by comparing the type of symptom of patient 1 and the type of symptom of patient 2. Furthermore, a health similarity score may be calculated by comparing the type of symptom of patient 1 and the underlying disease of patient 2. In this manner, a comprehensive health similarity score may be calculated by comparing all the items included in the health information of patient 1 with the items included in the health information of patient 2. The health similarity score may be calculated as an arithmetic value from 0% to 100%.

In one embodiment, when calculating the health similarity score, patients may be represented as n-dimensional data in a Euclidean space using their similar health information, and adjacent patients may be selected as patients with a health similarity score equal to or greater than the predetermined MDx similarity score, or may be classified according to a predetermined criterion in the Euclidean space. The algorithm for selecting patients with a health similarity score equal to or greater than the predetermined MDx similarity score is not limited to this, and various algorithms for regression or classification of conventionally disclosed data, such as K-Nearest Neighbor, SVM (Support Vector Machine), Decision Tree, and Greedy algorithm, may be used, but the invention is not limited thereto.

In FIG. 8, patient 1 and patient 3 have similar values for 'age', 'gender', 'type of symptom', 'degree of symptom', 'smoking status', 'alcohol consumption', 'height/weight', 'body fat percentage', 'body water content', and 'muscle mass', and slightly different 'underlying disease' and 'medications'. Therefore, a comprehensive health similarity score may be calculated when comparing the health information of patient 1 and patient 3 as a whole.

In this manner, it may be determined whether the health information is equal to or greater than a predetermined similarity in step S230 by comparing the health information of the target patient with the health information of previously stored patients.

In step S250, a patient whose health score is equal to or greater than a predetermined health similarity score may be selected. The patient selected in this way may be determined as a patient having health information similar to the target patient.

Referring back to step S300 of FIG. 2, the electronic medical record of the selected patient and the retrieved reference MDx data may be provided as the similarity case to the healthcare provider's terminal 200. The electronic medical record may include at least one of a symptom, an age, a gender, a vaccination history, medication information, an underlying disease name, and a lifestyle habit, while predefined sensitive information may be excluded from the health information included in the electronic medical record. The predefined sensitive information may include personal information. In one embodiment, only the parts of the electronic medical record necessary for treatment may be provided, and at least one element may be read-only or encrypted.

The electronic medical record of the selected patient may include the patient's symptoms, treatment information according to the MDx data, and information on the degree of improvement. The healthcare provider may use the provided electronic medical record and the retrieved reference MDx as auxiliary data to predict and determine the target patient's current state, a suitable treatment method for the target patient, and the degree of future improvement for the target patient.

In one embodiment, when the similarity case is provided to the healthcare provider's terminal, information about the compared items between the electronic medical record of the target patient and the electronic medical record of the patient may be provided together with the similarity case.

The similarity case may be classified with a matching rate of 0%~100% according to the MDx similarity score and the health similarity score. In one embodiment, the similarity case provided to the healthcare provider's terminal may correspond to 80%~100%, and the similarity case may be provided according to the matching rate desired by the healthcare provider's terminal.

At this time, information about the items compared in the health information to select a patient with the health similarity score equal to or greater than the predetermined health similarity score may be provided to the healthcare provider's terminal 200 along with the similarity case. By providing the compared items from the health information to the healthcare provider's terminal 200, the objectivity of the similarity case may be enhanced by presenting how suitable the similarity case data is for the target patient.

In one embodiment, an electronic medical record of a patient with the health similarity score less than the predetermined health similarity score may be provided as a dissimilarity case to the healthcare provider's terminal 200 by comparing the health information of the reference patient corresponding to the reference MDx data with the health information of the target patient. For example, it may be inferred that a patient has a similar symptom or is in a similar condition when corresponding to the reference MDx data, but a patient with a health similarity score less than the predetermined health similarity score, whose health information is not similar to that of the target patient, may be inferred to have a different symptom or condition from the target patient. Therefore, in this case, the electronic medical record or health information of the corresponding patient may be provided to the healthcare provider's terminal 200 as a dissimilarity case, which is an exceptional case for the target patient.

Similarly, when comparing the health information of a patient corresponding to MDx data less than the predetermined MDx similarity score, not the reference MDx data, with the health information of the target patient, when the patient with a health similarity score equal to or greater than the predetermined health similarity score exists, the electronic medical record of that patient may be provided as a dissimilarity case to the healthcare provider's terminal 200. That is, a patient corresponding to the MDx data with the MDx similarity score less than the predetermined MDx similarity score, which is not similar to the target patient's MDx data, may be inferred to have a dissimilar symptom or be in a dissimilar condition, but if a patient with the health similarity score equal to or greater than the predetermined health score, whose health information is similar to that of the target patient, exists, it may be inferred that their symptom or condition is similar to that of the target patient. Therefore, in this case, the electronic medical record or health information of the corresponding patient may be provided to the healthcare provider's terminal 200 as a dissimilarity case, which is an exceptional case for the target patient.

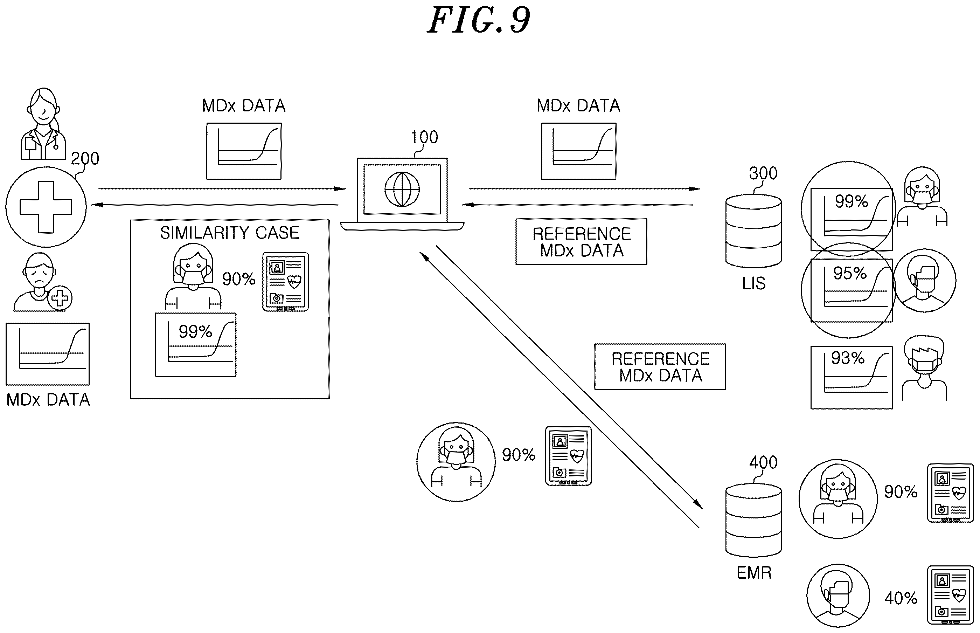

FIG. 9 is a diagram for explaining a structure in which the apparatus for providing the similarity case of the target patient using the MDx data 100 of FIG. 1 operates using an LIS server 300 and an EMR system 400.

In one embodiment, the apparatus for providing the similarity case 100 may include an internal database with pre-built MDx data, but it may also retrieve the MDx data using an external LIS (laboratory information system) server 300 and obtain the reference MDx data from the LIS DB.

At this time, the LIS server 300 may be a hospital or a testing institution, but is not limited thereto, and may refer to various servers that may store and manage MDx data and provide reference MDx data.

The apparatus for providing the similarity case 100 may transmit the MDx data obtained from the healthcare provider's terminal 200 to the LIS server 300, perform an operation to retrieve the reference MDx data with a predetermined similarity from the LIS server 300, and receive the reference MDx data from the LIS server 300.

In one embodiment, the apparatus for providing the similarity case 100 may include an internal database with pre-built electronic medical records and personal health information, but it may use an external EMR system 400 to compare the health information of the reference patient corresponding to the reference MDx data with the health information of the target patient to obtain the electronic medical record of a patient with a health similarity score equal to or greater than a predetermined health similarity score.

The apparatus for providing the similarity case 100 may transmit the reference MDx data obtained from the LIS server to the EMR system 400, or transmit the information corresponding to the patient in the reference MDx data to the EMR system 400. An operation of selecting a patient with the health similarity score equal to or greater than a predetermined health similarity score is performed in the EMR system 400, and the electronic medical record of the selected patient may be received from the EMR system 400.

So far, the operation of providing the similarity case of a target patient using MDx data has been described in detail with reference to FIGS. 2 to 8. Hereinafter, an exemplary computing apparatus 900 that may implement the apparatus for providing the similarity case 100 described in various embodiments will be described with reference to FIG. 9. In this embodiment, the computing apparatus 900 may be the apparatus for providing the similarity case 100 of FIG. 1.

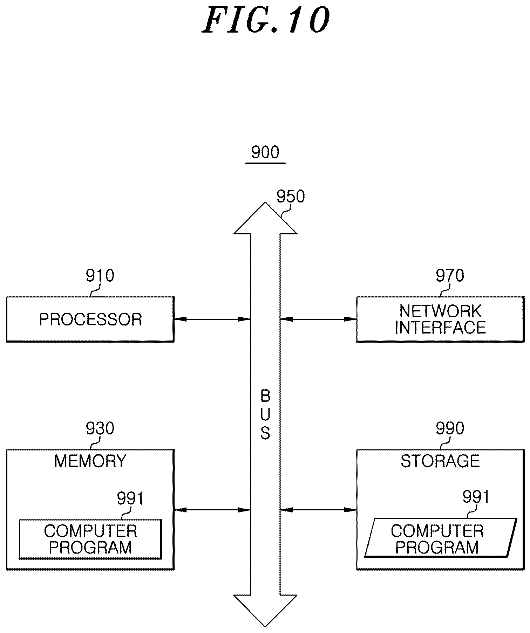

FIG. 10 is an exemplary hardware block diagram illustrating a computing apparatus 900.

As shown in FIG. 10, a computing apparatus 900 may include one or more processors 910, a bus 950, a network interface 970, a memory 930 that loads a computer program 991 to be executed by the processor 910, and a storage 990 that stores the computer program 991. However, FIG. 10 only shows components related to the embodiments of the present invention. Therefore, a person of ordinary skill in the art to which the present invention pertains will understand that other general-purpose components may be further included in addition to the components shown in FIG. 10.

The processor 910 controls the overall operation of each component of the computing apparatus 900. The processor 910 may be configured to include at least one of a Central Processing Unit (CPU), a Micro Processor Unit (MPU), a Micro Controller Unit (MCU), a Graphic Processing Unit (GPU), or any type of processor well known in the art. Furthermore, the processor 910 may perform operations for at least one application or program to execute the methods/operations according to various embodiments. The computing apparatus 900 may have one or more processors.

The memory 930 stores various data, instructions, and/or information. The memory 930 may load one or more programs 991 from the storage 990 to execute the methods/operations according to various embodiments of the present invention. For example, when the computer program 991 is loaded into the memory 930, a logic (or module) as shown in FIG. 4 may be implemented on the memory 930. An example of the memory 930 may be RAM, but is not limited to it.

The bus 950 provides a communication function between the components of the computing apparatus 900. The bus 950 may be implemented as various types of buses, such as an Address Bus, a Data Bus, and a Control Bus.

The network interface 970 supports wired and wireless internet communication of the computing apparatus 900. The network interface 970 may support various communication methods other than internet communication. For this purpose, the network interface 970 may be configured to include a communication module well known in the art.

The storage 990 may non-transitorily store one or more computer programs 991. The storage 990 may be configured to include non-volatile memory such as Read Only Memory (ROM), Erasable Programmable ROM (EPROM), Electrically Erasable Programmable ROM (EEPROM), flash memory, a hard disk, a removable disk, or any form of computer-readable recording medium well known in the art to which the present invention pertains.

The computer program 991 may include one or more instructions that implement the methods/operations according to various embodiments of the present invention. When the computer program 991 is loaded into the memory 930, the processor 910 may perform the methods/operations according to various embodiments of the present invention by executing the one or more instructions.

In one embodiment, the computer program 991 may include an instruction for retrieving the reference MDx data with the MDx similarity score above a predetermined MDx similarity score by comparing the target patient's MDx data with previously stored MDx data, an instruction for selecting a patient with a health similarity score above a predetermined health similarity score by comparing the health information of the reference patient corresponding to the retrieved reference MDx data with the health information of the target patient, and an instruction for providing the electronic medical record of the selected patient and the retrieved reference MDx data as the similarity case to the healthcare provider's terminal 200.

Meanwhile, the methods according to the various embodiments described above may be implemented in the form of a computer program stored on a computer-readable recording medium, programmed to perform each step of these methods, and may also be implemented in the form of a computer-readable recording medium that stores a computer program programmed to perform each step of these methods.

The above description is merely illustrative of the technical idea of the present disclosure, and those skilled in the art to which the present disclosure pertains will be able to make various modifications and variations without departing from the essential quality of the present disclosure. Accordingly, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

## Claims

1. A method for providing a similarity case of a target patient using molecular diagnostic (MDx) data, performed by an apparatus for providing the similarity case of the target patient, comprising:
obtaining reference MDx data for MDx data of the target patient by comparing the MDx data of the target patient with a previously stored set of MDx data;
obtaining an electronic medical record (EMR) of a reference patient(s) corresponding to health information of the target patient by comparing the health information of the target patient with health information of the reference patient from whom the reference MDx data are obtained; and
providing the obtained EMR and the obtained reference MDx data as the similarity case to a healthcare provider's terminal.

2. The method of claim 1, wherein the comparison of the MDx data of the target patient with the previously stored set of MDx data is performed by using a laboratory information system (LIS) storing the MDx data.

3. The method of claim 1, wherein the obtainment of the EMR is performed by using an EMR system storing the EMR.

4. The method of claim 1, wherein the MDx data are obtained from a plurality of target nucleic acid amplification reactions performed to detect a plurality of target nucleic acids of interest in a sample.

5. The method of claim 4, wherein the MDx data includes at least one of positive/negative determination results for the plurality of target nucleic acids of interest, Ct values for the plurality of target nucleic acids of interest, a dataset obtained from amplification reactions of the plurality of target nucleic acids of interest, and a value of a parameter indicating an aspect of the dataset.

6. The method of claim 4, wherein the reference MDx data are obtained by using at least one identical element among a reagent, an equipment, and an implementation method for the plurality of target nucleic acid amplification reactions for obtaining the MDx data.

7. The method of claim 6, wherein the identical equipment includes at least one selected from the group consisting of a nucleic acid extraction device, a liquid handler, and a real-time amplification device.

8. The method of claim 7, wherein the equipment has an identical calibration applied.

9. The method of claim 1, wherein the health information is extracted from content included in at least one of an EMR, a personal health record, and a health examination record.

10. The method of claim 1, wherein the health information includes at least one of a symptom, an age, a gender, a vaccination history, a medication information, an underlying disease, and a lifestyle habit.

11. The method of claim 1, wherein the electronic medical record provided to the healthcare provider's terminal includes at least one of a symptom, an age, a gender, a vaccination history, a medication information, an underlying disease name, and a lifestyle habit, and wherein the electronic medical record is exclusive of predefined sensitive information from health information included in the EMR, and
wherein the predefined sensitive information includes personal information.

12. The method of claim 1, wherein the reference patient corresponding to the health information of the target patient is selected by comparing at least one of the following between the target patient and the reference patient: a symptom, an age, a gender, a vaccination history, a medication information, an underlying disease name, and a lifestyle habit.

13. The method of claim 1, wherein said providing the obtained EMR and the obtained reference MDx data as the similarity case to the healthcare provider's terminal includes:
providing, to the healthcare provider's terminal, information about a compared item between the EMR of the reference patient and the EMR of the target patient, together with the similarity case.

14. The method of claim 1, wherein said providing the obtained EMR and the obtained reference MDx data as the similarity case to the healthcare provider's terminal includes:
providing, to the healthcare provider's terminal as the similarity case, a symptom of the reference patient, MDx data-based treatment information and post-treatment outcome information.

15. An apparatus for providing the similarity case of a target patient, comprising:
a processor;
a network interface;
a memory loading a computer program when executed by the processor; and
a storage storing the computer program,
wherein the computer program comprises:
an instruction for obtaining reference MDx data for MDx data of the target patient by comparing the MDx data of the target patient with a previously stored set of MDx data;
an instruction for obtaining an electronic medical record (EMR) of a reference patient(s) corresponding to health information of the target patient by comparing the health information of the target patient with health information of the reference patient from whom the reference MDx data are obtained; and
an instruction for providing the obtained EMR and the obtained reference MDx data as the similarity case to a healthcare provider's terminal.

16. The method of claim 15, wherein the MDx data are obtained from a plurality of target nucleic acid amplification reactions performed to detect a plurality of target nucleic acids of interest in a sample.

17. The method of claim 16, wherein the MDx data includes at least one of positive/negative determination results for the plurality of target nucleic acids of interest, Ct values for the plurality of target nucleic acids of interest, a dataset obtained from amplification reactions of the plurality of target nucleic acids of interest, and a value of a parameter indicating an aspect of the dataset.

18. The method of claim 15, wherein the reference MDx data are obtained by using at least one identical element among a reagent, an equipment, and an implementation method for the plurality of target nucleic acid amplification reactions for obtaining the MDx data.

19. The method of claim 15, wherein the health information is extracted from content included in at least one of an electronic medical record, a personal health record, and a health examination record.

20. The method of claim 15, wherein the health information includes at least one of a symptom, an age, a gender, a vaccination history, a medication information, an underlying disease, and a lifestyle habit.

21. A computer-readable recording medium storing a computer program programmed to perform:
obtaining reference MDx data for MDx data of a target patient by comparing the MDx data of the target patient with a previously stored set of MDx data;
obtaining an electronic medical record (EMR) of a reference patient(s) corresponding to health information of the target patient by comparing the health information of the target patient with health information of the reference patient from whom the reference MDx data are obtained; and
providing the obtained EMR and the obtained reference MDx data as a similarity case of the target patient to a healthcare provider's terminal.

22. A computer program stored in a computer-readable recording medium, wherein the computer program is programmed to perform:
obtaining reference MDx data for MDx data of a target patient by comparing the MDx data of the target patient with a previously stored set of MDx data;
obtaining an electronic medical record (EMR) of a reference patient(s) corresponding to health information of the target patient by comparing the health information of the target patient with health information of the reference patient from whom the reference MDx data are obtained; and
providing the obtained EMR and the obtained reference MDx data as a similarity case of the target patient to a healthcare provider's terminal.
